# EUROPEAN PATENT APPLICATION

(11) **EP 4 711 967 A1**
(43) Date of publication of application: **18.03.2026**
(21) Application number: 25201959.1
(22) Date of filing: 12.09.2025
(51) Int. Cl.: G06F 30/12, G06F 30/20, G16H 50/50

(54) **COMPUTER SYSTEM FOR COMPUTER AIDED DESIGN (CAD) MODELING OF A MITRAL VALVE**

(30) Priority: 13.09.2024 US 202418885013
(71) Applicant: Dassault Systemes Americas Corp., Waltham, MA 02451 (US)
(72) Inventor: YAO, Jiang, Waltham, MA 02451 (US); LIU, Hao, Waltham, MA 02451 (US); JIANG, Zhenxiang, Waltham, MA 02451 (US); BATTISTI, Thomas, Waltham, MA 02451 (US)
(74) Representative: Fish & Richardson P.C.

(57) **Abstract**

Systems and method for generating a patient specific CAD model of a mitral valve include receiving digital images of a mitral valve of a patient and segmenting the digital images to identify structures of the mitral valve. A CAD model of the mitral valve is generated including modeled structures representing the identified structures. The modeled structures in the CAD model are connected at multiple locations. First loading conditions for the modeled structures are determined using a first hemodynamics model. Movement of the modeled structures are simulated based on the first loading conditions. A specified area is determined based on the CAD model. Second loading conditions are determined using a second hemodynamics model that receives the specified area as an input. The CAD model is calibrated by modifying a configuration of the modeled structures in the CAD model based on the loading conditions and movement of the modeled structures.

## Description

### TECHNICAL FIELD

This description generally relates to generating a CAD model of a mitral valve.

### BACKGROUND

In general, patient specific models include anatomical or physiological models of a patient or a portion of a patient that will interact with a medical device that can be used in treatment of a physical condition of the patient. For example, a patient specific model can be used to assess a device fit or a device design for improved performance of the treatment.

### SUMMARY

Mitral regurgitation (MR) is a form of heart disease in which the mitral valve does not properly close when a patient's heart pumps blood. MR results in a backwards flow of blood from the left ventricle through the mitral valve into the left atrium when the left ventricle contracts. One method of treating MR includes a transcatheter edge-to-edge treatment where a clip is attached to each leaflet of the mitral valve to hold the leaflets together. The mitral valve can continue to open and close in areas not attached to the clip.

This disclosure provides an approach to generate a patient specific model of a mitral valve that can be used to determine the effectiveness of a transcatheter edge-to-edge repair. The patient specific model can be a multi-physics model that includes both the structure of the patient's mitral valve represented in a computer-aided design (CAD) model of the mitral valve and blood flow through the mitral valve represented by a lumped parameter hemodynamics model. The patient specific model can be calibrated to match physiological and anatomical parameters measured from the patient in a pre-operative state. A CAD model of a clip can be coupled to the CAD model of the mitral valve to simulate the dynamics of the patient's mitral valve in a post-operative state. The selection of the type of clip, the number of clips, and the positioning of the clips can be adjusted to improve the post-operative outcomes. The patient can be treated by inserting the clip into the patient's heart (e.g., through a catheter), and attaching the clip to the patient's mitral valve based on the simulated post-operative state of the patient specific model.

In an example implementation, a data processing system (e.g., a computer system with one or more processors) generates a patient specific model of a mitral valve. The data processing system segments digital images (e.g., computed-tomography (CT) scans) of the patient's mitral valve to isolate structures of the mitral valve (e.g., leaflets, chordae, annulus, papillary muscles). The data processing system generates a CAD model of the mitral valve based on the isolated structures. The CAD model includes data representing the isolated structures. The data processing system connects modeled chordae to modeled leaflets at multiple locations. The data processing system extends the modeled annulus in the CAD model to represent fibrous tissue around the circumference of the mitral valve. The data processing system determines initial loading conditions on the modeled structures in the CAD model using a stand-alone lumped parameter hemodynamics model that simulates blood flow through the patient's heart. The data processing system performs a co-simulation of the mitral valve using the CAD model, an anatomic orifice area module that determines an anatomic orifice area of the CAD model, and a coupled lumped parameter hemodynamics model that takes as input the anatomic orifice area and outputs leaflet pressures to apply to the leaflets in the CAD model. The data processing system calibrates the CAD model by modifying the configuration of the modeled chordae and the modeled leaflets based on the leaflet pressures determined from the coupled lumped parameter hemodynamics module and the movement of the modeled structures in the CAD model in comparison to the isolated structures from the segmentation.

The implementations described herein can provide various technical benefits. As an example, the implementations described herein allow a computer system to represent a specific geometry and performance of a specific patient's mitral valve to enable customized treatment of the patient's MR. The approach of this disclosure generates a patient specific model that more closely represents the actual physiological and anatomical conditions of the patient's mitral valve as compared to previous models. The patient specific model of this disclosure can be used to evaluate whether a specific patient may benefit from having the treatment performed. The computer system can output clinically relevant metrics for MR assessment and treatment (e.g., MR grade, regurgitant flow, etc.). As another example, the implementations described herein can be used to perform in silico clinical trials that assess the efficacy of a proposed treatment by applying the treatment to multiple patient specific models.

In an aspect, a method for generating a patient specific CAD model of a mitral valve includes receiving, by a data processing system, one or more digital images of a mitral valve of a patient; segmenting, by the data processing system, the one or more digital images to identify structures of the mitral valve; generating, by the data processing system, a CAD model of the mitral valve, the CAD model comprising data for modeled structures representing the identified structures of the mitral valve; connecting, by the data processing system in the CAD model, one or more first ones of the modeled structures to one or more second ones of the modeled structures and third ones of the modeled structures, the one or more first ones connecting to the one or more second ones and third ones at multiple locations; determining, by the data processing system, one or more first loading conditions for the modeled structures in the CAD model using a first hemodynamics model; simulating, by the data processing system, movement of the modeled structures in the CAD model based on the first loading conditions applied to the modeled structures; determining, by the data processing system, a specified area based on the CAD model; determining, by the data processing system, one or more second loading conditions using a second hemodynamics model that receives data representing the specified area as an input; and calibrating, by the data processing system, the CAD model by modifying a configuration of one or more first ones of the modeled structures and one or more second ones of the modeled structures in the CAD model, with the modifying based on (i) the one or more second loading conditions determined using the second hemodynamics model, and (ii) positions of the modeled structures in the CAD model based on the movement of the modeled structures in the CAD model in comparison to positions of the identified structures.

Implementations of this aspect can include one or more of the following features.

In some implementations, segmenting the one or more digital images includes rotating the one or more digital images to align with an annulus plane of the mitral valve.

In some implementations, the identified structures include an annulus, leaflets, papillary muscles, and chordae.

In some implementations, segmenting the one or more digital images includes verifying a geometry of the leaflets across leaflets identified in multiple digital images.

In some implementations, the modeled structures include a modeled annulus, modeled leaflets, modeled papillary muscles, and modeled chordae based on the identified structures, and the one or more first ones include the modeled chordae, the one or more second ones include the model leaflets, the one or more third ones include the modeled papillary muscles, and the fourth one includes the modeled annulus.

In some implementations, connecting the one or more first ones of the modeled structures to the one or more second ones and the one or more third ones includes clustering the modeled chordae into circular zones on each modeled leaflet, each circular zone corresponding to a location of the multiple locations on the modeled papillary muscles.

In some implementations, calibrating the CAD model includes determining an initial chordae length for the modeled chordae based on a thermal analysis of the CAD model in a systole condition and in a diastole condition; simulating movement of the modeled structures in the CAD model by iteratively determining modeled leaflet positions, orifice areas for the modeled leaflet positions, and pressures applied to the modeled leaflets, the pressures being determined using the second hemodynamics model and the orifice areas; determining a distance error between the simulated movement and a position of the mitral valve in the one or more digital images; and in response to determining that the distance error exceeds a threshold distance error, adjusting geometries of the modeled leaflets, a number of the modeled chordae, chordae attachment locations of the modeled chordae, or modeled chordae lengths to correct the distance error.

Some implementations include extending, by the data processing system, the modeled annulus in the CAD model to represent tissue in association with the mitral valve.

In some implementations, the first and the second hemodynamics models include first and second lumped parameter hemodynamics models.

In some implementations, generating the CAD model includes generating modeled leaflet geometry at a diastole condition of the mitral valve, and generating modeled leaflet geometry at a systole condition of the mitral valve.

Some implementations include treating a patient based on performing pre-operative simulations and post-operative simulations using the CAD model of the mitral valve.

In some implementations, performing post-operative simulations using the CAD model includes placing a model of a medical device in contact with one or more of the modeled structures in the CAD model of the mitral valve.

In some implementations, the model of the medical device includes a model of a clip in contact with the modeled structures of the mitral valve in the CAD model.

In some implementations, the specified area includes defining two or more parallel cut planes in the CAD model; forming one or more connected segments; determining a coaptation point and a coaptation length on each of the two or more parallel cut planes based on the one or more connected segments; and determining the specified area based on the coaptation length and a distance between two parallel cut planes of the two or more parallel cut planes.

In some implementations, the first and the second hemodynamics models include parameters determined by minimizing a difference between outputs of the first and the second hemodynamics models and corresponding measurements from the patient and literature data.

Some implementations include generating clinical metrics for use in assessment of mitral valve regurgitation in pre-operative and post-operative states of treated patients, where the clinical metrics include left atrial pressure, transmitral pressure gradient, and mitral valve regurgitant volume.

Other embodiments of this aspect include corresponding computer systems, apparatuses, and computer programs recorded on one or more computer storage devices, each configured to perform the actions or operations described herein. A system of one or more computers can be configured to perform particular actions by virtue of having software, firmware, hardware, or a combination of them installed on the system that in operation causes the actions or causes the system to perform the actions. One or more computer programs can be configured to perform particular actions by virtue of including instructions that, when executed by a data processing apparatus, cause the apparatus to perform the actions.

The details of one or more embodiments of the subject matter of this specification are set forth in the accompanying drawings and the description below. Other features, aspects, and advantages of the subject matter will become apparent from the description, the drawings, and the claims.

### BRIEF DESCRIPTION OF THE DRAWINGS

FIG. 1 is a block diagram of an example system for CAD modeling of a mitral valve.
FIG. 2 is a flowchart for an example process for generating a patient specific model of a mitral valve.
FIG. 3 illustrates an example segmentation of digital images of a patient's mitral valve at multiple times during a cardiac cycle.
FIG. 4 is a block diagram of an example lumped parameter hemodynamics model.
FIGS. 5A and 5B illustrate top and perspective views of an example patient specific CAD model of a mitral valve in a diastole condition.
FIG. 5C illustrates the patient specific CAD model of FIGS. 5A-5B in a systole condition.
FIG. 6 is a composite plot illustrating an example process for determining an anatomic orifice area at multiple conditions of a patient specific mitral valve.
FIG. 7 illustrates example modeled chordae attachment locations on modeled leaflets of a mitral valve.
FIG. 8 is a flowchart of an example process for calibrating a patient specific CAD model of a mitral valve.
FIGS. 9A and 9B depict an example CAD model of a clip for a mitral valve.
FIG. 9C depicts the clip of FIGS. 9A-9B attached to a CAD model of a mitral valve.
FIGS. 10A-10D illustrate an example CAD model of a mitral valve in diastole and systole conditions for both a pre-operative state and a post-operative state.
FIG. 11 is a flowchart for an example process for generating a patient specific model of a mitral valve.
FIG. 12 is a diagram of an example computer system.

Like reference numbers and designations in the various drawings indicate like elements.

### DETAILED DESCRIPTION

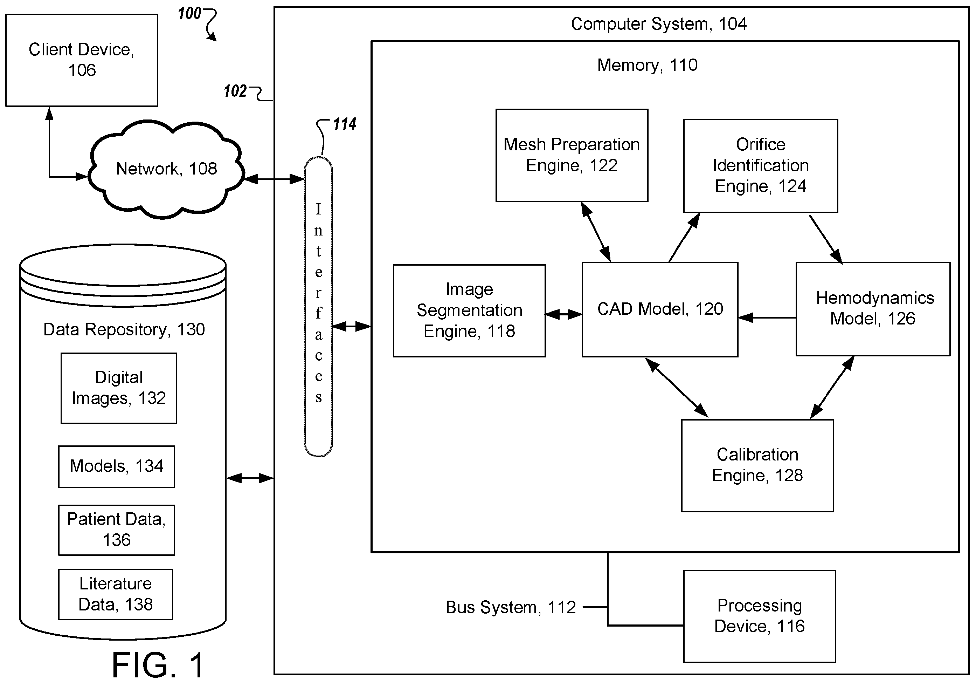

FIG. 1 is a block diagram of an example system 100 for CAD modeling of a mitral valve or mitral valve apparatus (MVA). The MVA can include the mitral valve annulus, the mitral valve leaflets, the papillary muscles, and chordae tendineae. The system 100 in this implementation is based on a client-server or cloud-based architecture and includes a server system 102 implemented as a computer system 104 (stand alone or cloud-based) and a client device 106 coupled via a network 108. The server system 102 includes memory 110, a bus system 112, interfaces 114 (e.g., user interfaces/network interfaces/display or monitor interfaces, etc.) and a processing device 116. In memory 110, are an image segmentation engine 118, a CAD model 120, a mesh preparation engine 122, an orifice identification engine 124, a hemodynamics model 126, and a calibration engine 128. The system 100 also includes a data repository 130 that includes digital images 132, models 134, patient data 136, and literature data 138.

The image segmentation engine 118 receives the digital images 132 from the data repository 130. The image segmentation engine 118 identifies structures of the MVA in the digital images 132. For example, the image segmentation engine 118 determines pixels of the digital images that correspond to structures of the MVA (e.g., annulus, leaflets, chordae, papillary muscles), and pixels that do not correspond to the structure (e.g., blood pixels).

The CAD model 120, the orifice identification engine 124, and the hemodynamics model 126 form a multi-physics, patient specific model that includes data representing the physical structure of the MVA and blood flow through the MVA. The CAD model 120, orifice identification engine 124, and hemodynamics model 126 are coupled together to perform multi-physics simulations of the MVA (e.g., in a co-simulation). For example, the CAD model 120 can be used in structural simulations to determine leaflet positions of the mitral valve in response to applied boundary conditions such as pressure applied to leaflets and positional data applied to the annulus region. The structural simulation can determine the positions of the modeled leaflets using an incremental approach. For example, the structural simulation can determine a next position of the modeled leaflets by advancing the solution using small time steps without iteration within each increment. The leaflet positions are sent to the orifice identification engine 124 where the orifice in the mitral valve can be identified. The orifice identification engine 124 can determine an orifice area of the mitral valve based on the leaflet positions of the CAD model 120 (e.g., the area through which blood can flow). The orifice identification engine 124 sends the identified orifice (e.g., the orifice area) to the hemodynamics model 126 where loading conditions on the CAD model 120 (e.g., leaflet pressures, transmitral pressure differences) can be determined based on blood flow through the heart using the identified orifice as an input. The loading conditions are sent to the CAD model 120 to be applied as boundary conditions in a subsequent iteration of the simulation.

The CAD model 120 includes data representing the structure and geometry of the MVA of a patient. For example, the CAD model 120 includes data representing the location and orientation of structures of the MVA such as leaflets, chordae attached to papillary muscles and the leaflets, and an annulus region. The CAD model 120 also includes structural properties (e.g., shape, thickness, leaflet fibers, configuration and cross-sectional area of chordae, etc.) and material properties (e.g. anisotropy, inhomogeneity, hyperelasticity, density, etc.). The geometry of the CAD model 120 can be specified at one or more states during the cardiac cycle. The cardiac cycle includes a full heartbeat (e.g., from the start of the heartbeat to the start of the next heartbeat). The cardiac cycle includes a diastole phase (e.g., ventricular filling) and a systole phase (e.g., ventricular contraction/ejection).

The CAD model 120 is incorporated into a structural simulation (e.g., a finite element model) to determine movement of the elements of the CAD model 120 in response to applied boundary conditions (e.g., pressures acting on leaflets, displacements of annulus and papillary muscles to match observed positions of the patient's annulus and papillary muscles, etc.). For example, the structural simulation can solve the three-dimensional, time resolved equations derived from the principle of virtual work. This approach provides equilibrium between the internal and external forces for virtual displacements enabling accurate simulation of the solid mechanics of the MVA.

The mesh preparation engine 122 can determine a mesh to apply to the CAD model 120 in the structural simulations. The mesh can be a discretized representation of the CAD model 120 with volumetric elements (e.g., cells, voxels, tetrahedral elements, or hexahedral elements) representing the modeled structures in the CAD model 120. While FIG. 1 shows mesh preparation engine 122 in memory 110, the mesh preparation engine can be a third-party application that is executed on a different system than server system 102. Whether mesh preparation engine 122 executes in memory 110 or is executed on a different system than server 102, mesh preparation engine 122 receives a user-supplied mesh definition based on the CAD model 120 and then prepares a mesh and sends (and/or stores) the prepared mesh for use in structural simulations (e.g., finite element simulations) of the CAD model 120.

In some implementations, the CAD model 120 includes models of both mitral valve leaflets and idealized chordae. The papillary muscles may not be modeled; however, the coordinates of the papillary-chordae attachment locations are defined in the CAD model 120. The leaflets can be modelled with a uniform thickness (e.g., approximately 2 millimeters). The leaflets can be discretized using linear, incompatible mode, hexahedral brick elements (e.g., by the mesh preparation engine 122). The leaflet tissue can be simulated using an anisotropic Holzapfel-Ogden constitutive law. Material constants for the CAD model 120 can be determined using data from the literature (e.g., biaxial human leaflet tensile data). The chordae can be modelled with a uniform-cross sectional area and discretized using truss elements. The material response of the chordae can be modelled using an Ogden hyperelastic constitutive law. Each chordae can be considered as a single line of elements (e.g., no branching) between the origin point on a modeled papillary muscle and the insertion point on the modeled leaflet.

The papillary-chordae attachment locations and the annular edges of the leaflets can be prescribed as boundary conditions (either time-varying or fixed boundary conditions). In some implementations, general surface-to-surface contacts can be frictionless to account for collisions between opposing leaflets and among elements within the same leaflet.

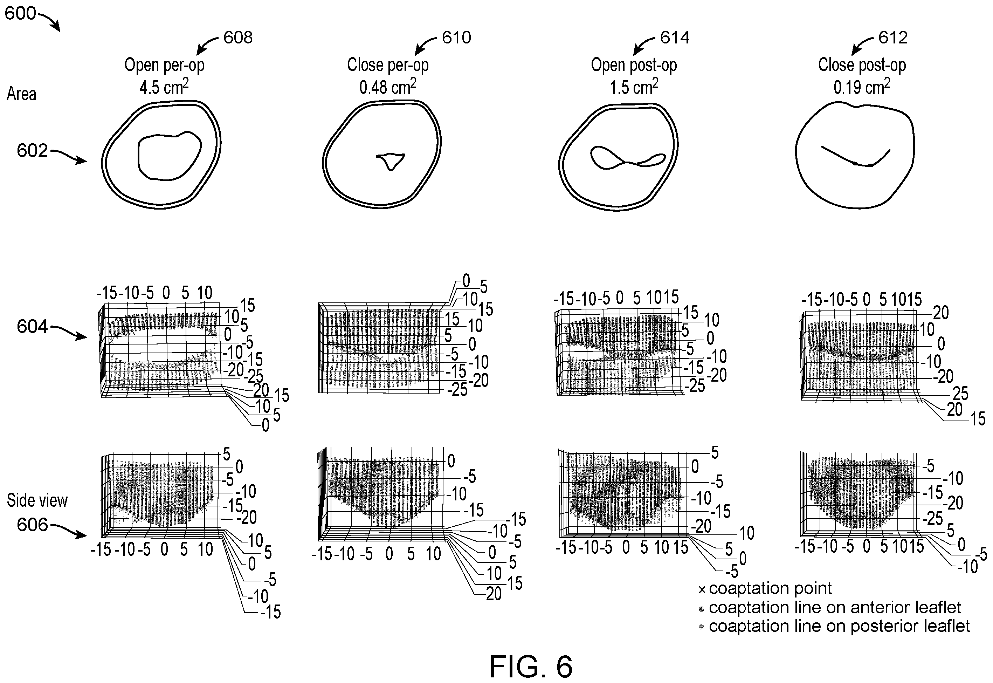

The orifice identification engine 124 receives the mesh of the MVA from the CAD model 120. The orifice identification engine 124 identifies an orifice in the received mesh. The orifice identification engine can determine the orifice area through which blood can flow through the mitral valve as represented by the positions of the modeled structures in the CAD model. The orifice area changes throughout the cardiac cycle. Consequently, the orifice area can be determined at multiple times during the cardiac cycle to reflect the amount of area through which blood can flow at the respective times in the cardiac cycle. The orifice identification engine 124 can be called at multiple time steps of the structural simulation of the CAD model 120 (e.g., every timestep, every second timestep, every fifth timestep, every tenth time step, times determined based on phases of the cardiac cycle, etc.). An example process that can be implemented by the orifice identification engine 124 to determine the orifice area is discussed in further detail in reference to FIG. 6.

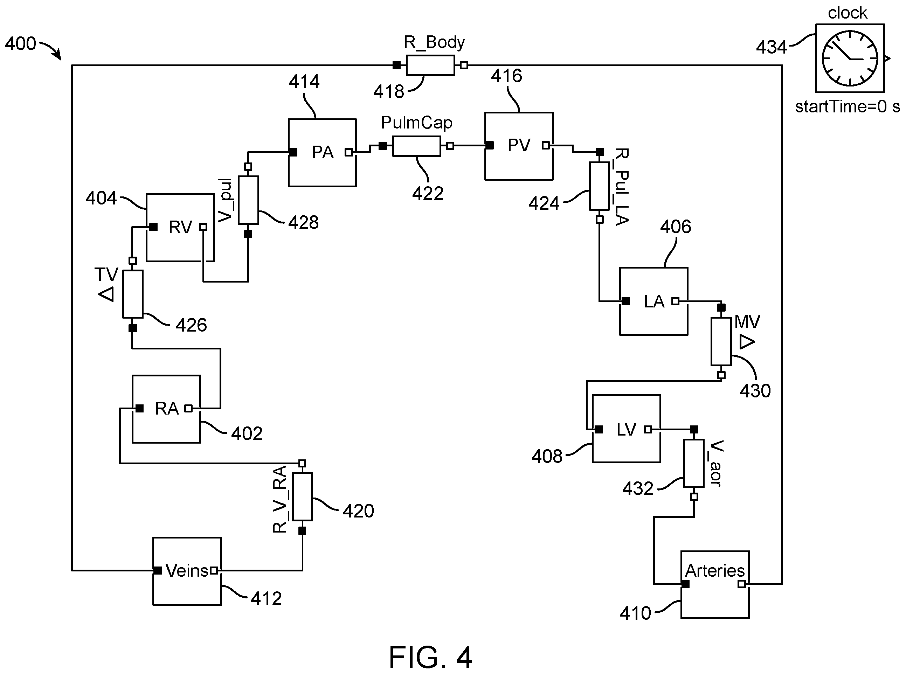

The hemodynamics model 126 simulates blood flow through the heart of the patient. The hemodynamics model 126 can be a closed-loop lumped parameter model (LPM) that predicts pressure boundary conditions on the MVA (e.g., transmitral pressure difference). The hemodynamics model 126 simulates the bulk effects of blood flow within the heart to and from the circulatory system. Flow between the ventricles and atria through the mitral and tricuspid valves can be modeled using a Bernoulli-like relationship that can capture the effects of blood inertance and blood resistance. The hemodynamics model 126 can receive the orifice identified by the orifice identification engine 124 as an input. The orifice area of the identified orifice is related to the pressures applied to the leaflets in the CAD model 120. For example, relatively large orifice areas (as compared with the total mitral valve area) correspond to lower pressure gradients exerted on the leaflets by the blood flow during the diastole phase. However, larger orifice areas correspond to higher regurgitation during the systole phase. The hemodynamics model 126 can produce as outputs, the loading conditions for the CAD model 120 (e.g., leaflet pressures) for a subsequent time step of the structural simulation. An example lumped parameter hemodynamics model is discussed in further detail in reference to FIG. 4.

The calibration engine 128 is used to tailor the CAD model 120 and the hemodynamics model 126 to match specific clinical metrics from a patient and specific geometry of the MVA of the patient. The calibration engine 128 can adjust a configuration of modeled structures in the CAD model 120 based on positions of the modeled structures during simulated movements of the modeled structures in the CAD model 120 in comparison to identified structures from the image segmentation engine 118. The calibration engine 128 can adjust parameters of the hemodynamics model to match clinically measured patient data 136 and literature data 138. After calibration, the CAD model 120 and the hemodynamics model 126 represent a pre-operative state of a patient. The system 100 can store the CAD model 120 and the hemodynamics model 126 in the data repository for retrieval and use at a later time. For example, the computer system 104 can access the models 134 for use in a device treatment simulation of the MVA.

The system 100 can produce clinically relevant output parameters that can be used to assess treatment viability and efficacy. Example clinical outputs include regurgitant volume, regurgitant fraction, effective orifice area, left atrial pressure, mitral valve orifice area, diastolic mitral valve pressure gradient, and diastolic left ventricle volume and left ventricle ejection fraction.

The system 100 can simulate blood flow through the mitral valve in a pre-operative state of the patient using the CAD model 120, the orifice identification engine 124, and the hemodynamics model 126 throughout the cardiac cycle. For example, the simulation can start at 70% diastole. Initial chamber pressures are specified (e.g., based on literature data 138, patient data 136, and flow equations). The nodes in the CAD model 120 at the modeled annulus region and the modeled papillary muscle attachment points are fixed (e.g., based on image segmentation data). The modeled chordae lengths are adjusted at diastole to achieve an approximate systolic state where the modeled chordae are straight. Resulting strain in the modeled chordae and the modeled leaflets are set to zero (e.g., to approximate a nearly strain-free state at 70% diastole). After adjustment of the modeled chordae and the modeled leaflets, a co-simulation using the CAD model 120, the orifice identification engine 124 and the hemodynamics model 126 can begin. The hemodynamics model 126 determines loading conditions (e.g., pressures) to apply to the modeled leaflets in the CAD model 120 using the orifice area determined by the orifice identification engine 124. During the co-simulation, the annular and attachment node locations in the CAD model 120 that were previously fixed are prescribed to be time-varying. The system 100 simulates multiple cardiac cycles until reaching a steady-state response.

Example inputs of the structural simulation of the CAD model 120 include leaflet material parameters, chordae material parameters, chordae length, leaflet size and shape, annulus size, shape and motion, and chordae-papillary attachment locations and motions. Example outputs of the structural simulation of the CAD model 120 include motion and shape of geometric components, coaptation behavior (e.g., height, tenting area, posterior leaflet angle), stress and strain in leaflets and chordae, mitral valve orifice area, and regurgitant orifice area and locations. Other inputs and outputs of the structural simulation of the CAD model 120 are possible.

Example inputs of the hemodynamics model 126 include initial chamber pressures and volumes, chamber passive compliances, chamber active elastances, flow model parameters through mitral and tricuspid valves (e.g., resistance, inertance, blood density), flow resistances through pulmonary and aortic valves, and flow resistances through chambers (except valves). Example outputs of the hemodynamics model 126 include chamber pressure and volume traces, left ventricular ejection fraction, left ventricular systolic and diastolic volumes, systolic and diastolic transmitral pressure gradients, regurgitant volume and fraction, pulmonary veinous waveforms, and mean atrial pressure. Other inputs and outputs of the hemodynamic model 126 are possible.

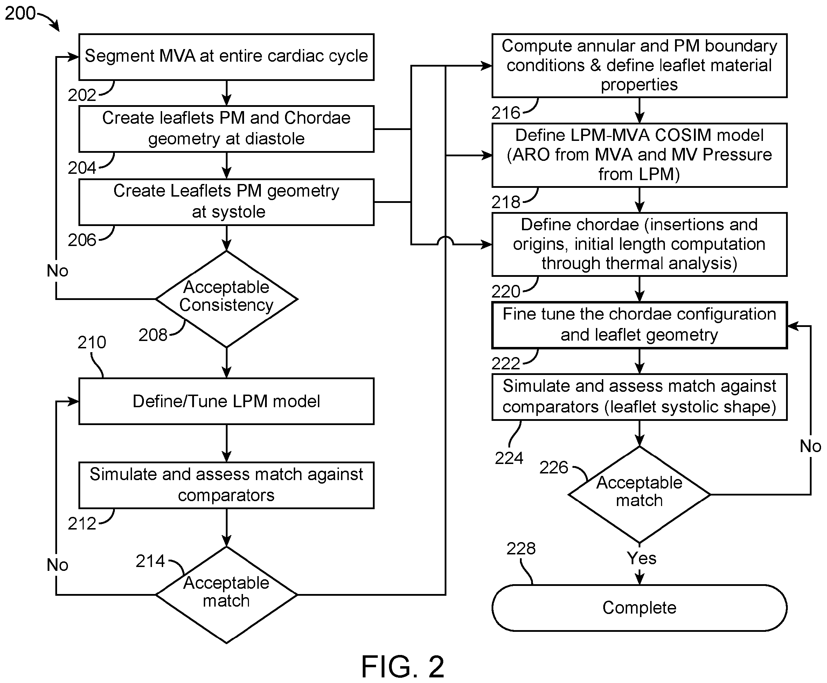

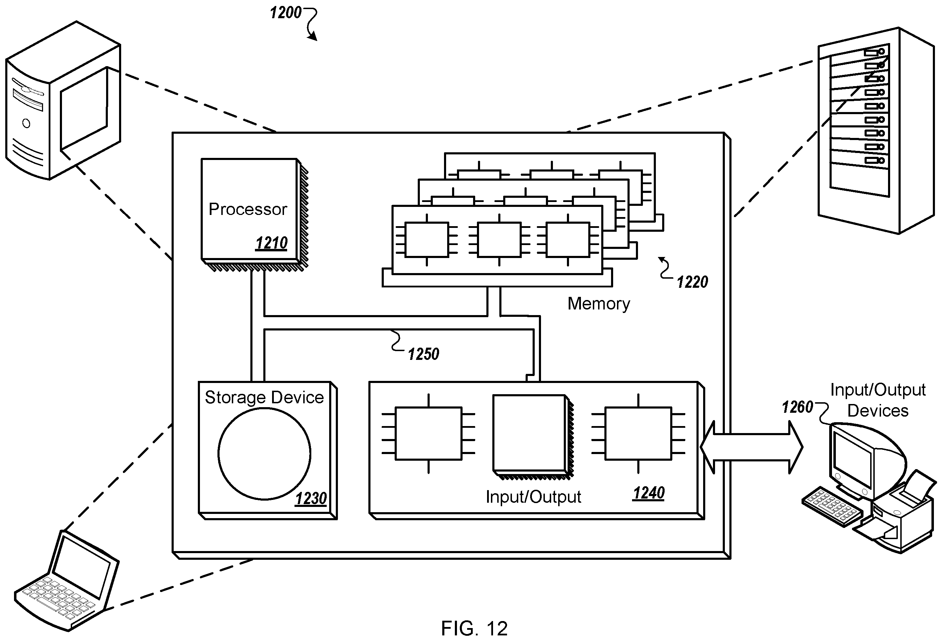

FIG. 2 is a flowchart for an example process 200 for generating a patient specific model of an MVA. The process 200 can be implemented on a data processing system (e.g., the computer system of FIG. 12). The process 200 includes generating a CAD model of a patient's MVA (steps 202-208), generating a stand-alone lumped parameter hemodynamics model (steps 210-214) to determine initial loading conditions for a structural simulation of the CAD model, and calibrating the CAD model to match comparators through co-simulation with a second lumped parameter hemodynamics model (steps 216-226). The process 200 is complete 228 when the patient specific model represents a pre-operative condition of the patient.

The CAD model of the patient's MVA is generated based on isolated structures identified in three-dimensional (3D) digital images (e.g., CT, MRI, ultrasound scans including transesophageal echocardiography and transthoracic echocardiography). The data processing system segments the MVA, including mitral valve leaflets, annulus, and papillary muscles, in the images during an entire cardiac cycle (step 202). To segment the images, the data processing system rotates the images to adjust the leaflet orientation to clearly view the commissure location (e.g., the location where the anterior and posterior leaflets come together at their insertion into the annulus). The images can be rotated to align the image plane with the annulus plane of the mitral valve. The data processing system can segment the annulus, anterior and posterior leaflets, papillary muscles, and chordae to isolate these structures. The data processing system can perform the segmentation at defined intervals during the cardiac cycle.

The data processing system creates modeled leaflets, modeled papillary muscles, and modeled chordae geometry in the CAD model at a diastole condition (step 204). For example, the data processing system determines the 3D position of the leaflets, papillary muscles, and chordae based on the isolated structures from the segmentation. The data processing system creates idealized geometry of the modeled structures in the diastole condition based on the determined 3D positions of the isolated structures in the diastole condition.

The data processing system creates modeled leaflets, modeled papillary muscles, and modeled chordae geometry in the CAD model at a systole condition (step 206). For example, the data processing system determines the 3D position of the leaflets, papillary muscles, and chordae based on the isolated structures from the segmentation. The data processing system creates idealized geometry of the modeled structures in the systole condition based on the determined 3D positions of the isolated structures in the systole condition.

The data processing system compares the CAD model at the diastole condition and the CAD model at the systole condition with the image segmentation to determine if the generated CAD model has an acceptable level of consistency with the images (step 208). If there is not acceptable consistency, the process 200 returns to the segmentation of the MVA (step 202).

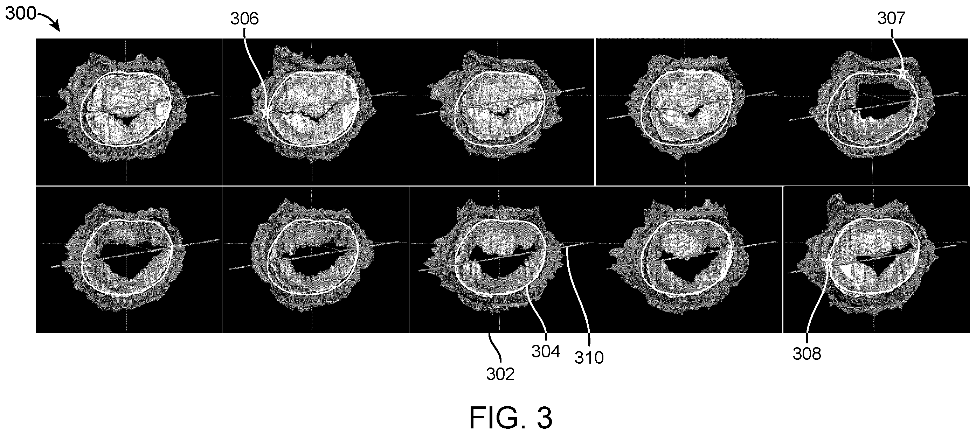

Turning briefly to FIG. 3, FIG. 3 shows a series of frames 300 visualizing segmented structures of the MVA throughout the cardiac cycle. In the eight frame 302, an outline of the annulus 304 is drawn. The outline of the annulus 304 is overlaid on the other frames 300 to assess consistency of the segmentation and modeled geometry. The data processing system identifies inconsistencies 306, 307, and 308 where a mismatch between the outline of the annulus 304 and the modeled geometry occurs. The modeled geometry and the outline of the annulus 304 are aligned using the line 310 that extends between the two commissure points. After identifying the inconsistencies 306, 307, and 308, the data processing system modifies the modeled geometries to resolve the inconsistencies.

Returning to FIG. 2, the data processing system defines an LPM (step 210) to represent hemodynamics of the blood flow through the heart of the patient. The data processing system utilizes the LPM to determine initial loading conditions to apply to the CAD model during calibration of the CAD model. The LPM defined in step 210 is a stand-alone LPM that uses anatomic orifice area based on an approximation. For example, the hemodynamics of the valve can be determined using the Mynard model (Mynard, J.P., Davidson, M. R., Penny, D.J., Smolich, J.J., "A simple, versatile valve model for use in lumped parameter and one-dimensional cardiovascular models," International Journal of Numerical Methods in Biomedical Engineering (2012), incorporated by reference herein in its entirety). A second LPM is generated at step 218 for use in the co-simulation that instead uses the anatomic orifice area based on the CAD model.

The data processing system simulates blood flow through the heart using the LPM and compares outputs of the LPM with comparators (step 212). The outputs of the LPM include pressures and volumes of heart chambers, pressure differences across heart valves, MR volumes, etc. The comparators include patient data that can be measured corresponding to the LPM outputs. In some implementations, patient data is unavailable, and the comparators can be based on data reported in literature.

The data processing system determines if the LPM is an acceptable match with the comparators (step 214). If the LPM is acceptable, the process 200 proceeds to the co-simulation definition. If the match is unacceptable, the process 200 reverts to step 210 and the input parameters of the LPM are tuned to better match the comparators. For example, the data processing system can tune the LPM model parameters using an optimization algorithm that seeks to minimize the mismatch between the LPM outputs and the comparators. Constraints on the optimization can include physiological ranges of the input parameters. The data processing system repeats steps 210-214 until an acceptable match is achieved.

Example LPM outputs that the data processing system can seek to match with patient measurements include left ventricular ejection fraction (LV EF), left ventricular end diastolic volume (LV ED vol), left ventricular end systolic volume (LV ES vol), left atrial end diastolic volume (LA ED vol), left atrial end systolic volume (LV ES vol), left atrial mean pressure, left atrial V wave pressure, mitral stenosis, and MR severity.

FIG. 4 is a block diagram of an example LPM 400. The LPM 400 represents bulk blood flow within the heart and bulk blood flow to and from the circulatory system. The LPM 400 models the active chambers 402-408 of the heart (right atrium 402, right ventricle 404, left atrium 406, left ventricle 408) as active capacitors with time-varying capacitance. The time-varying capacitance simulates the elastances of the ventricular and atrial chambers of the heart. The LPM 400 includes passive capacitors representing arteries 410, veins 412, the pulmonary artery 414 and the pulmonary vein 416. The LPM 400 includes several flow nodes 418-424 (body 418, veins to right ventricle 420, pulmonary capillary 422, and pulmonary vein to left atrium 424). The flow nodes 418-424 are modeled as resistors that linearly relate pressure gradient and blood flow. The heart valves 426-432 (tricuspid valve 426, pulmonary valve 428, mitral valve 430, aortic valve 432) are modeled as resistors providing resistance to the blood flow in the heart. The tricuspid valve 426 and mitral valve 430 can be modeled based on the Mynard model, and the pulmonary valve 428 and the aortic valve 432 can be modeled as one-way flow nodes (e.g., allow flow in only one direction).

The LPM 400 includes a timer 434 that represents the timestep of the model. Blood flow is initiated in the LPM 400 in response to initial pressure differences assigned to the active chambers 402-408. Blood flow between the ventricles and atria through the tricuspid valve 426 and the mitral valve 430 can be modeled using a Bernoulli-like equation that captures the effect of blood inertance, blood resistance, and time-varying orifice area, where the orifice area is determined based on the Mynard model.

The LPM 400 can output pressure and volume histories for the duration of the simulation for the active chambers 402-408 and passive chambers 410-416. The LPM 400 can output flow rates and flow time integrals (e.g., volume of blood flow) for the valves 426-432.

Returning to FIG. 2, at step 216, the data processing system determines annular and papillary muscle boundary conditions. The annular and papillary muscle boundary conditions can be determined based on the leaflet and papillary muscle geometry created at steps 204 and 206.

The data processing system extends the modeled annulus region as a stiffer material to represent fibrous tissue around the circumference of the mitral valve. The data processing system applies the boundary conditions to the extended annulus structure rather than directly to the modeled leaflets. For example, the data processing system can extend the modeled annuls region be creating a line that is tangent to a curve positioned 3 mm away from the modeled annulus region for each point on the edge of the annulus. The points can be connected into a spline which the data processing system can close off to form a volume. The data processing system can connect nodes (e.g., nodes of the mesh) in the extended annulus region to ground nodes using an element representing a spring. The data processing system can apply displacement boundary conditions to the ground nodes which transmit the displacement to the nodes in the extended annulus region through the elements representing springs. Applying the displacement boundary conditions to the extended annulus region through the ground nodes and spring elements removes kinematic over-constraint of the nodes in the extended annulus region. The data processing system can adjust the spring stiffness to achieve accurate motion of the modeled annulus region.

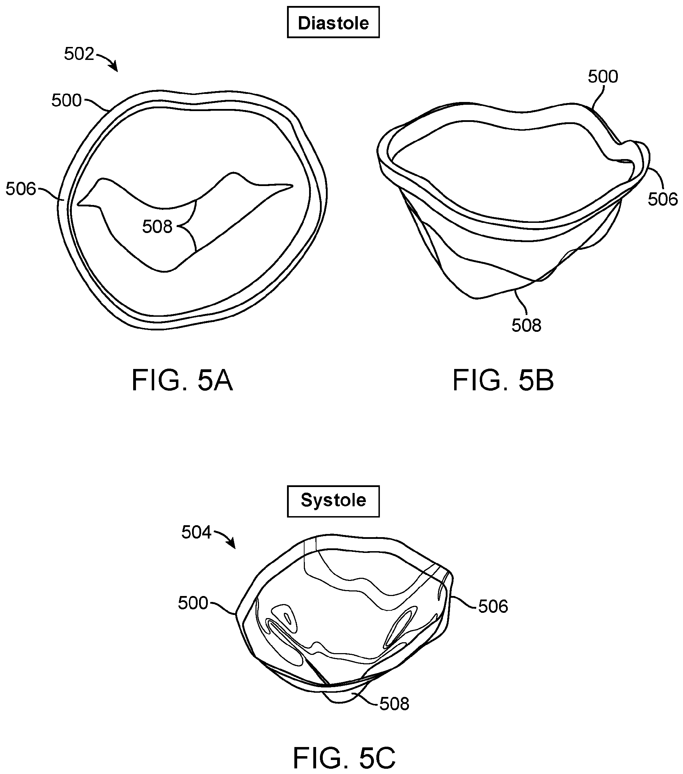

Briefly, FIGS. 5A and 5B illustrate top and perspective views of an example patient specific CAD model 500 of a MVA in a diastole condition 502. FIG. 5C illustrates the patient specific CAD model 500 in a systole condition 504. The CAD model includes an extended annulus region 506 that represents fibrous tissue around the MVA. In structural simulations using the CAD model 500, boundary conditions can be applied to the extended annulus region 506 rather than directly to the leaflets 508.

Returning to FIG. 2, the data processing system defines a co-simulation model based on the CAD model, an LPM, and an anatomic orifice area module. The LPM used in the co-simulation model is substantially the same as the stand-alone LPM except the LPM of the co-simulation model receives the orifice area for the mitral valve from the anatomic orifice area module rather than approximating the orifice area. The other input parameters of the LPM that were tuned to match comparators remain the same.

The anatomic orifice area module determines the orifice area based on the CAD model. The anatomic orifice area module defines parallel cut planes in the CAD model. Each cut plane intersects the leaflets on one or more connected segments. A connected segment is a continuous curve on the leaflet. The anatomic orifice area module determines one or more potential coaptation points on each connected segment. A potential coaptation point can only coapt with another potential coaptation point on a different connected segment. When a coaptation pair is identified (e.g., a coaptation point on each of two segments in a cut plane), the superior point is considered as the coaptation point. The closest distance to the opposing connected segment defines the coaptation length for that cut plane. The anatomic orifice area is determined based on the sum of the coaptation lengths and the distance between adjacent cut planes.

FIG. 6 is a composite plot 600 illustrating coaptation lines and points used to determine an anatomic orifice area at multiple conditions of a patient specific MVA model. The top row 602 shows a top view of the mesh of the CAD model of the MVA. The middle row 604 shows the top view of the coaptation lines along the cut planes. The bottom row shows a side view 606 of the coaptation lines along the cut planes. The first column 608 shows an open valve in a pre-operative state. The second column 610 shows a closed valve in the pre-operative state. The third column 612 shows an open condition of a valve in a post-operative state. The fourth column 614 shows a closed condition of the valve in the post-operative state. The anatomic orifice area module defines the parallel cut planes in the CAD model. The anatomic orifice area module defines coaptation lines and coaptation points for each cut plane.

Returning to FIG. 2, the data processing system defines modeled chordae in the CAD model (step 220). The modeled chordae connect the modeled papillary muscle locations with the modeled leaflets taking into account the anatomy of the papillary muscle based on the image segmentation. During segmentation, the data processing system segments the chordae at the diastole frame and the papillary muscles at each frame processed. While not all chordae are visible in the digital images, modeled chordae are placed in the CAD model based on the location and orientation of the visible chordae. The data processing system defines additional chordae in the CAD model (e.g., chordae that are not visible in the digital images) according to physiologically ranges of chordae.

The data processing system places chordae into the CAD model in accordance with the anatomy of the mitral valves and papillary muscles. There are two groups of papillary muscles: anterior papillary muscles (APM) and posterior papillary muscles (PPM). Each group of papillary muscles includes 13 origin locations. For each half of the leaflet (one half connected to APM and the other half connected to PPM), the chordae insertion points are clustered into circular zones. Several chordae insertions are located in each zone (e.g., 5-6 insertions). The data processing system connects the modeled chordae in each circular zone with a particular papillary muscle origin location. The data processing system explicitly defines the connection between the circular zone and the papillary muscle origin location. For example, the data processing system identifies the papillary muscle origin location for the modeled chordae based on proximity between the modeled leaflet insertion location and/or based on geometry identified in the segmentation and digital images.

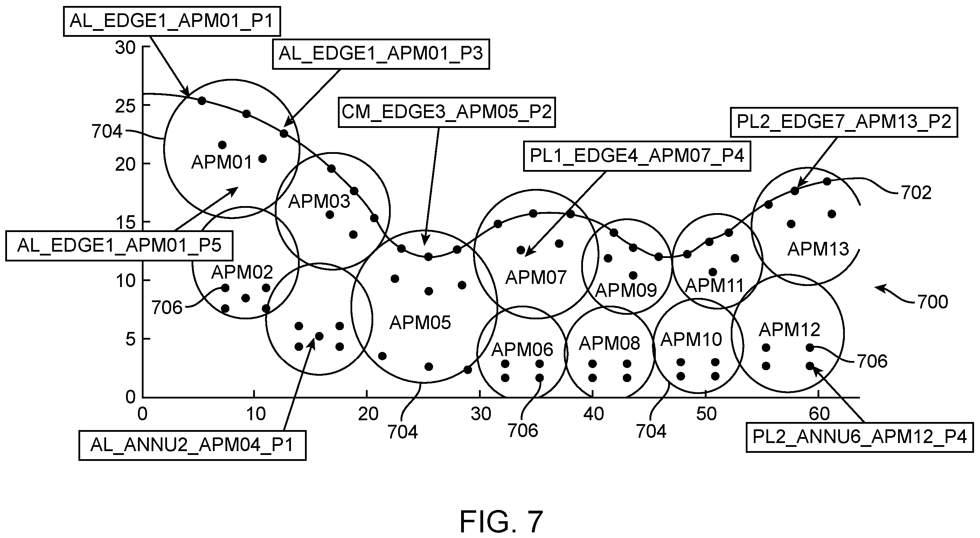

FIG. 7 illustrates an example half leaflet 700 with modeled chordae insertion locations. The half leaflet 700 has been flattened with the curved free edge 700 of the leaflet 700 oriented on top. Several circular zones 704 are defined along the leaflet 700 with modeled chordae insertion locations 706 within each zone connected to a particular papillary muscle origin location. Some of the chordae insertion locations 706 are defined on the curved edge 702. The insertion locations 706 are non-homogenously distributed across the leaflet 700. Defining the chordae insertion locations 706 in this manner can result in a larger mitral valve orifice area in the CAD model at diastole when a patient is treated using a device that clips the mitral valve which better represents observed clinical behaviors.

Turning back to FIG. 2, the data processing system fine tunes the chordae configuration and leaflet geometry (step 222). The data processing system can modify the length and tension of the modeled chordae. Additionally, the data processing system can modify the modeled leaflet length. Modifying the configuration of the modeled chordae and the modeled leaflets can result in a better match of the systolic shape and regurgitation of blood through the mitral valve.

The data processing system simulates movement of the MVA and blood flow through the mitral valve using the co-simulation model, and the data processing system assesses the modeled chordae and modeled leaflets to determine a match against comparators (e.g., systolic shape) (step 224). The data processing system determines the acceptability of the match with the comparators (step 226). If the determined match is unacceptable, the data processing system modifies the configuration of the modeled chordae and the modeled leaflets to achieve a better match with the comparators (e.g., between a systolic condition from the structural simulation of the CAD model and the image segmentation). The data processing system can repeat steps 222-226 until an acceptable match is achieved.

The process 200 is complete 228 when the CAD model has an acceptable match with the comparators. When the process 200 is complete 228, the data processing system stores the patient specific model in a hardware storage device for use in subsequent (e.g., occurring later) simulations and/or rendering visualizations of the patient specific model for display on a display device. Visualizations can include, for example, animations of the MVA in one or more phases of the cardiac cycle as determined by movement of modeled structures in the CAD model and clinical metrics determined from the simulation (e.g., blood flow rates, pressures, etc.).

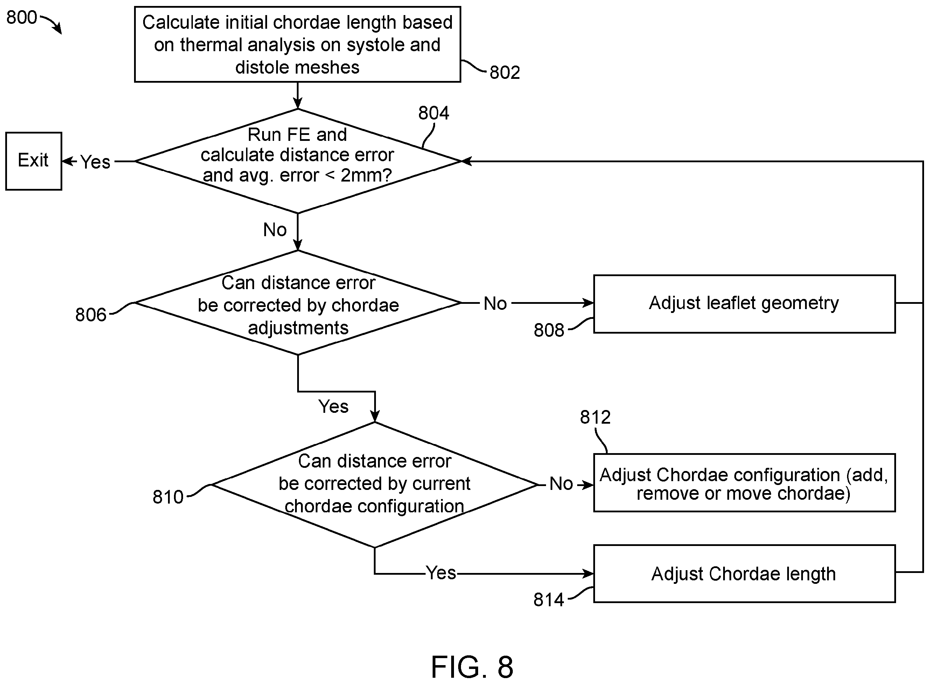

FIG. 8 is a flowchart of an example process 800 for calibrating a patient specific CAD model of an MVA. The process 800 can be implemented by a data processing system to modify the configuration of modeled chordae and modeled leaflets in the CAD model to better match comparators from image segmentation, patient data, and/or literature data. For example, the data processing system can implement the process 800 to modify the modeled chordae and modeled leaflets while performing steps 220-226 of the process 200.

The process 800 begins with the data processing system calculating initial chordae lengths for the modeled chordae based on thermal analysis of the CAD model of the MVA in systole and diastole conditions (step 802). After setting the initial chordae lengths in the CAD model, the data processing system performs a structural simulation of the movement of the modeled MVA and determines a distance error between the simulated movement and positions of the MVA determined from image segmentation (step 804). If the distance error is below a threshold value (e.g., if the average distance error is less than 2 mm), then the process 800 is complete. Otherwise, the data processing system determines if the distance error can be corrected by chordae adjustments (step 806). For example, distance errors caused by tenting and billowing responses in the modeled leaflets can be corrected by chordae adjustments. Distance errors caused by excessive or insufficient lengths of the modeled leaflets can be corrected by adjusting the lengths of the modeled leaflets.

If the distance error exceeds the threshold value and cannot be corrected with chordae adjustments, the data processing system modifies the modeled leaflet geometry (step 808). For example, the data processing system can adjust the lengths of the modeled leaflets. After adjusting the modeled leaflets, the data processing system returns to step 804 to rerun the structural simulation and determine the distance error.

If the distance error exceeds the threshold value and can be corrected with chordae adjustments, the data processing system determines if the distance error can be corrected with the current chordae configuration (step 810).

If the distance error can be corrected with the current chordae configuration, the data processing system adjusts the chordae length to resolve the distance error (step 814). For example, the data processing system can adjust the initial chordae length of the modeled chordae based on the determined distance error. Additionally, or alternatively, the data processing system can adjust the pretension of the modeled chordae attached to the modeled leaflets. The data processing system can adjust the modeled chordae pretension based on mismatches between the structural simulations and the image segmentation in the systole condition. For example, the data processing system can adjust the commissure chordae pretension to reduce openings at the commissure. After adjusting the lengths of the modeled chordae, the data processing system returns to step 804 to rerun the structural simulation and determine the distance error.

If the distance error cannot be corrected with the current chordae configuration, the data processing system modifies the chordae configuration (step 812). For example, the data processing system can add or remove modeled chordae from the CAD model. Additionally, or alternatively, the data processing system can move the chordae insertion/attachment locations on the modeled leaflets. After adjusting the configuration of the modeled chordae, the data processing system returns to step 804 to rerun the structural simulation and determine the distance error.

The data processing system can repeat steps 804-812 until the distance error is below the threshold value distance error. In some implementations, the data processing system stops iterations once a set number of iterations has been reached and the distance error still exceeds the threshold value of the distance error. In such implementations, the data processing system can generate an error message indicating that the maximum number of iterations has been reached and the distance error exceeds the threshold value.

The processes 200 and 800 generate a patient specific MVA model in a pre-operative condition. For example, the patient specific MVA model reflects the current state of the patient's MVA before any intervention has been performed. The patient specific model can then be used to determine the effectiveness of performing an intervention to resolve MR. For example, a clip can be inserted into the patient's heart and attached to the mitral valve leaflets to alleviate symptoms of MR. To simulate the effectiveness of such a treatment, a CAD model of a clip can be deployed within the patient specific MVA model. The data processing system can then perform the co-simulation using the combined structural CAD model, a lumped parameter hemodynamics model, and the anatomic orifice area module to determine clinical metrics useful to a medical professional in determining potential effectiveness of the treatment. This co-simulation can be performed iteratively, and the selection of the type of clip, the number of clips, and/or the positioning of the clip on the mitral valve leaflets can be adjusted between iterations to find a position that best reduces regurgitation through the mitral valve while maintaining an acceptable transmitral pressure gradient.

Based on the results of the treatment simulations using the patient specific model, the patient can be treated by inserting the clip into the heart of the patient and attaching the clip to the patient's mitral valve. The clip can be inserted using catheters without requiring open heart surgery.

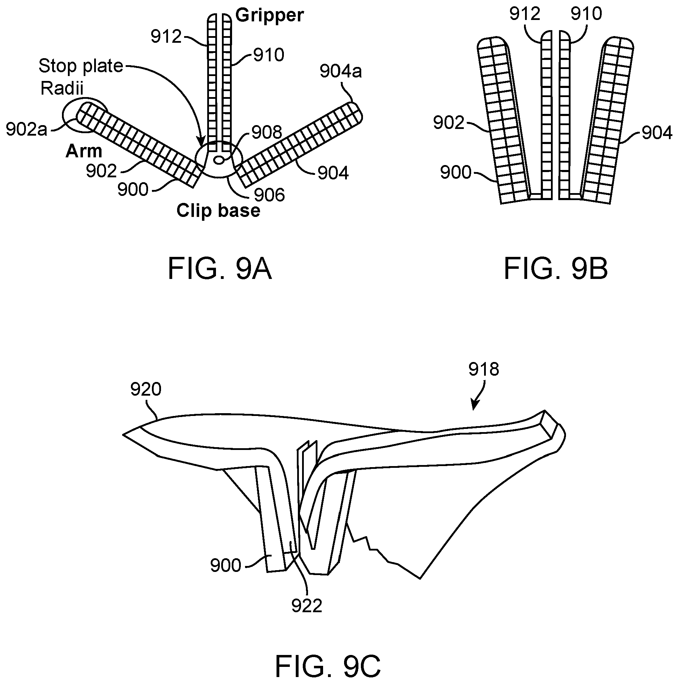

FIGS. 9A and 9B depict an example CAD model of a clip 900 for a mitral valve. FIG. 9A shows the clip 900 in an open position. FIG. 9B shows the clip 900 in a closed configuration. The clip 900 includes two arms 902, 904 connected at the clip base 906. The arms 902, 904 have rounded ends 902a, 904a.. A stop plate 908 is located near the clip base 906 to prevent the leaflets from sliding through the base of the clip 900. The clip 900 also includes two grippers 910, 912. When installed on the mitral valve, the leaflets are inserted between the arms 902, 904 and the respective grippers 912, 910. After insertion of the leaflets, the clip 900 is closed to attach to the leaflets. During a simulation, no slip boundary conditions can be implemented between the arms 902, 904, the grippers 912, 910, and the modeled leaflets after the leaflets are fully inserted into the arms to prevent relative motion between the clip 900 and the mitral valve leaflets (effectively bonding the leaflets against the arms and grippers). The clip 900 can be allowed to move with the mitral valve during the simulation upon closure (e.g., allowed to move in six degrees of freedom).

FIG. 9C depicts a coupled CAD model 918 including the CAD model of the clip 900 attached to a CAD model of a MVA 920. The coupled CAD model 918 can be used to simulate clip deployment and post-operative MR. The clip 900 holds the leaflets 922 of the CAD model 918 in a closed position to reduce MR. The coupled CAD model (including the clip 900 and the MVA 920) can be included as the CAD model in the co-simulation of the MVA. Example additional inputs specific to the coupled CAD model include clip deployment translation and rotation, rotations of clip grippers and arms. Example additional outputs specific to the coupled CAD model include clipping forces and moments prior to clip release, clipping length, grip pressure and pattern, and post-clipping regurgitant orifice area, shape, and locations.

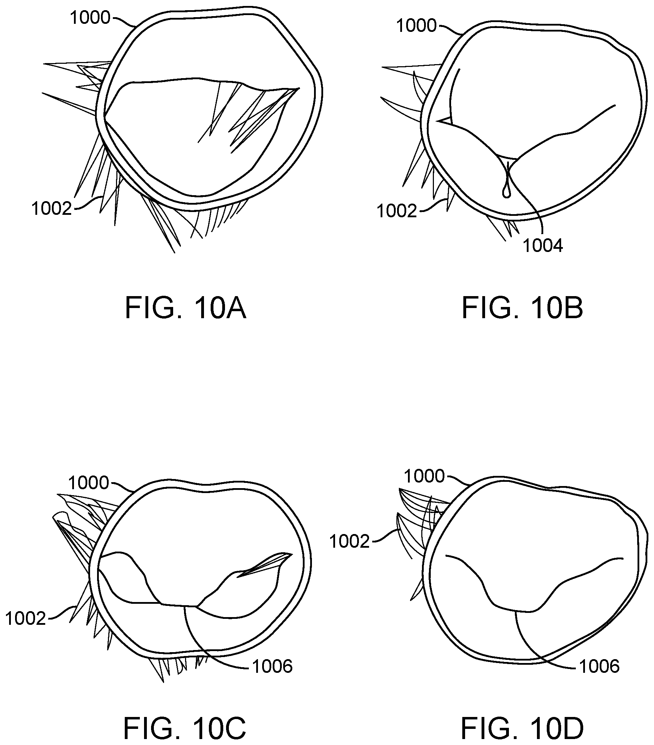

FIGS. 10A-10D illustrate an example CAD model of a MVA 1000 with modeled chordae 1002 in diastole and systole conditions for both a pre-operative state and a post-operative state. FIGS. 10A-10B show the pre-operative conditions in the diastole and systole conditions, respectively. In the systole condition, openings 1004 in the mitral valve are still present allowing regurgitation through the mitral valve 1000. FIGS. 10C-10D show the post-operative condition in the diastole and systole conditions, respectively. The clip location 1006is seen in the post-operative diastole condition (FIG. 10C) and produces a clinically observed double orifice allowing ventricle filling. In the systole condition (FIG. 10D), the opening and hence mitral regurgitation has been significantly reduced.

Multiple patient specific models of MVAs can be generated (a virtual patient cohort) using physical data or synthetically generated data. The results of the treatment simulations can be used to predict effectiveness for the cohort. During design and testing of medical devices and treatments, in Silico clinical trials performed on a virtual patient cohort can be useful for predicting patient outcomes and reducing costs and patient risks associated with a new treatment. In Silico clinical trials can also be used to determine enrollment criteria for physical clinical trials to prospectively exclude patients less likely to respond favorably to an intervention.

### Example Processes

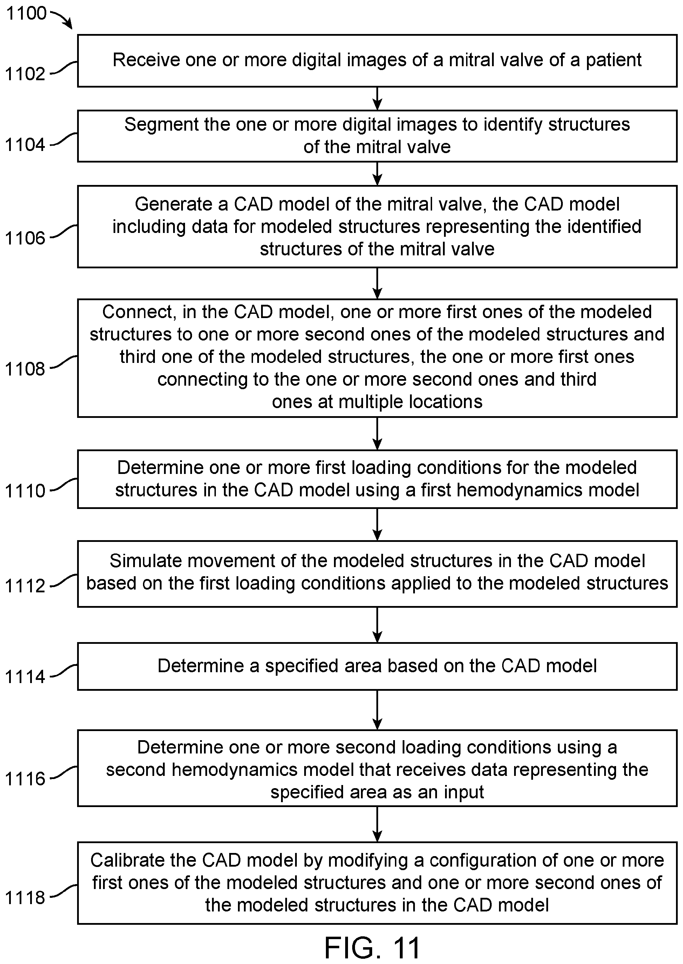

FIG. 11 shows an example process 1100 for generating a CAD model of a mitral valve. In some implementation, the process 1100 can be performed by the system 100 described in this disclosure.

In the process 1100, a system receives 1102 one or more digital images of a mitral valve of a patient. The system segments 1104 the one or more digital images to identify structures of the mitral valve. The system generates 1106 a CAD model of the mitral valve. The CAD model includes data for modeled structures representing the identified structures of the mitral valve. The system connects 1108, in the CAD model, one or more first ones of the modeled structures to one or more second ones of the modeled structures and third ones of the modeled structures. The one or more first ones connect to the one or more second ones and third ones at multiple locations. The system determines 1110 one or more first loading conditions for the modeled structures in the CAD model using a first hemodynamics model. The system simulates 1112 movement of the modeled structures in the CAD model based on the first loading conditions applied to the modeled structures. The system determines 1114 a specified area based on the CAD model. The system determines 1116 one or more second loading conditions using a second hemodynamics model that receives data representing the specified area as an input. The system calibrates 1118 the CAD model by modifying a configuration of one or more first ones of the modeled structures and one or more second ones of the modeled structures in the CAD model, with the modifying based on (i) the one or more second loading conditions determined using the second hemodynamics model, and (ii) positions of the modeled structures in the CAD model based on the movement of the modeled structures in the CAD model in comparison to positions of the identified structures.

In some implementations, segmenting the one or more digital images includes rotating the one or more digital images to align with an annulus plane of the mitral valve.

In some implementations, the identified structures include an annulus, leaflets, papillary muscles, and chordae.

In some implementations, segmenting the one or more digital images includes verifying a geometry of the leaflets across leaflets identified in multiple digital images.

In some implementations, the modeled structures include a modeled annulus, modeled leaflets, modeled papillary muscles, and modeled chordae based on the identified structures, and the one or more first ones include the modeled chordae, the one or more second ones include the model leaflets, the one or more third ones include the modeled papillary muscles, and the fourth one includes the modeled annulus.

In some implementations, connecting the one or more first ones of the modeled structures to the one or more second ones and the one or more third ones includes clustering the modeled chordae into circular zones on each modeled leaflet, each circular zone corresponding to a location of the multiple locations on the modeled papillary muscles.

In some implementations, calibrating the CAD model includes determining an initial chordae length for the modeled chordae based on a thermal analysis of the CAD model in a systole condition and in a diastole condition; simulating movement of the modeled structures in the CAD model by iteratively determining modeled leaflet positions, orifice areas for the modeled leaflet positions, and pressures applied to the modeled leaflets, the pressures being determined using the second hemodynamics model and the orifice areas; determining a distance error between the simulated movement and a position of the mitral valve in the one or more digital images; and in response to determining that the distance error exceeds a threshold distance error, adjusting geometries of the modeled leaflets, a number of the modeled chordae, chordae attachment locations of the modeled chordae, or modeled chordae lengths to correct the distance error.

In some implementations, the system extends the modeled annulus in the CAD model to represent tissue in association with the mitral valve.

In some implementations, the first and the second hemodynamics models include first and second lumped parameter hemodynamics models.

In some implementations, generating the CAD model includes generating modeled leaflet geometry at a diastole condition of the mitral valve, and generating modeled leaflet geometry at a systole condition of the mitral valve.

Some implementations include treating a patient based on performing pre-operative simulations and post-operative simulations using the CAD model of the mitral valve.

In some implementations, performing post-operative simulations using the CAD model includes placing a model of a medical device in contact with one or more of the modeled structures in the CAD model of the mitral valve.

In some implementations, the model of the medical device includes a model of a clip in contact with the modeled structures of the mitral valve in the CAD model.

In some implementations, the specified area includes defining two or more parallel cut planes in the CAD model; forming one or more connected segments; determining a coaptation point and a coaptation length on each of the two or more parallel cut planes based on the one or more connected segments; and determining the specified area based on the coaptation length and a distance between two parallel cut planes of the two or more parallel cut planes.

In some implementations, the first and the second hemodynamics models include parameters determined by minimizing a difference between outputs of the first and the second hemodynamics models and corresponding measurements from the patient and literature data.

In some implementations, the system generates clinical metrics for use in assessment of mitral valve regurgitation in pre-operative and post-operative states of treated patients, where the clinical metrics include left atrial pressure, transmitral pressure gradient, and mitral valve regurgitant volume.

### Example Computer Systems

FIG. 12 depicts an example computing system, according to implementations of the present disclosure. The system 1200 may be used for any of the operations described with respect to the various implementations discussed herein. The system 1200 may include one or more processors 1210, a memory 1220, one or more hardware storage devices 1230, and one or more input/output (I/O) devices 1260 controllable through one or more I/O interfaces 1240. The various components 1210, 1220, 1230, 1240, or 1260 may be interconnected through at least one system bus 1250, which may enable the transfer of data between the various modules and components of the system 1200.

The processor(s) 1210 may be configured to process instructions for execution within the system 1200. The processor(s) 1210 may include single-threaded processor(s), multi-threaded processor(s), or both. The processor(s) 1210 may be configured to process instructions stored in the memory 1220 or on the storage device(s) 1230. The processor(s) 1210 may include hardware-based processor(s) each including one or more cores. The processor(s) 1210 may include general purpose processor(s), special purpose processor(s), or both.

The memory 1220 may store information within the system 1200. In some implementations, the memory 1220 includes one or more computer-readable media. The memory 1220 may include any number of volatile memory units, any number of non-volatile memory units, or both volatile and non-volatile memory units. The memory 1220 may include read-only memory, random access memory, or both. In some examples, the memory 1220 may be employed as active or physical memory by one or more executing software modules.

The storage device(s) 1230 may be configured to provide (e.g., persistent) mass storage for the system 1200. In some implementations, the storage device(s) 1230 may include one or more computer-readable media. For example, the storage device(s) 1230 may include a floppy disk device, a hard disk device, an optical disk device, or a tape device. The storage device(s) 1230 may include read-only memory, random access memory, or both. The storage device(s) 1230 may include one or more of an internal hard drive, an external hard drive, or a removable drive.

One or both of the memory 1220 or the storage device(s) 1230 may include one or more computer-readable storage media (CRSM). The CRSM may include one or more of an electronic storage medium, a magnetic storage medium, an optical storage medium, a magneto-optical storage medium, a quantum storage medium, a mechanical computer storage medium, and so forth. The CRSM may provide storage of computer-readable instructions describing data structures, processes, applications, programs, other modules, or other data for the operation of the system 1200. In some implementations, the CRSM may include a data store that provides storage of computer-readable instructions or other information in a non-transitory format. The CRSM may be incorporated into the system 1200 or may be external with respect to the system 1200. The CRSM may include read-only memory, random access memory, or both. One or more CRSM suitable for tangibly embodying computer program instructions and data may include any type of non-volatile memory, including but not limited to: semiconductor memory devices, such as EPROM, EEPROM, and flash memory devices; magnetic disks such as internal hard disks and removable disks; magneto-optical disks; and CD-ROM and DVD-ROM disks. In some examples, the processor(s) 1210 and the memory 1220 may be supplemented by, or incorporated into, one or more application-specific integrated circuits (ASICs).

The system 1200 may include one or more I/O devices 1260. The I/O device(s) 1260 may include one or more input devices such as a keyboard, a mouse, a pen, a game controller, a touch input device, an audio input device (e.g., a microphone), a gestural input device, a haptic input device, an image or video capture device (e.g., a camera), or other devices. In some examples, the I/O device(s) 1260 may also include one or more output devices such as a display, LED(s), an audio output device (e.g., a speaker), a printer, a haptic output device, and so forth. The I/O device(s) 1260 may be physically incorporated into one or more computing devices of the system 1200 or may be external with respect to one or more computing devices of the system 1200.

The system 1200 may include one or more I/O interfaces 1240 to enable components or modules of the system 1200 to control, interface with, or otherwise communicate with the I/O device(s) 1260. The I/O interface(s) 1240 may enable information to be transferred in or out of the system 1200, or between components of the system 1200, through serial communication, parallel communication, or other types of communication. For example, the I/O interface(s) 1240 may comply with a version of the RS-232 standard for serial ports, or with a version of the IEEE 1284 standard for parallel ports. As another example, the I/O interface(s) 1240 may be configured to provide a connection over Universal Serial Bus (USB) or Ethernet. In some examples, the I/O interface(s) 1240 may be configured to provide a serial connection that is compliant with a version of the IEEE 1394 standard.

The I/O interface(s) 1240 may also include one or more network interfaces that enable communications between computing devices in the system 1200, or between the system 1200 and other network-connected computing systems. The network interface(s) may include one or more network interface controllers (NICs), or other types of transceiver devices configured to send and receive communications over one or more networks using any network protocol.

Computing devices of the system 1200 may communicate with one another, or with other computing devices, using one or more networks. Such networks may include public networks such as the internet, private networks such as an institutional or personal intranet, or any combination of private and public networks. The networks may include any type of wired or wireless network, including but not limited to local area networks (LANs), wide area networks (WANs), wireless WANs (WWANs), wireless LANs (WLANs), mobile communications networks (e.g., 3G, 4G, Edge, etc.), and so forth. In some implementations, the communications between computing devices may be encrypted or otherwise secured. For example, communications may employ one or more public or private cryptographic keys, ciphers, digital certificates, or other credentials supported by a security protocol, such as any version of the Secure Sockets Layer (SSL) or the Transport Layer Security (TLS) protocol.

The system 1200 may include any number of computing devices of any type. The computing device(s) may include, but are not limited to: a personal computer, a smartphone, a tablet computer, a wearable computer, an implanted computer, a mobile gaming device, an electronic book reader, an automotive computer, a desktop computer, a laptop computer, a notebook computer, a game console, a home entertainment device, a network computer, a server computer, a mainframe computer, a distributed computing device (e.g., a cloud computing device), a microcomputer, a system on a chip (SoC), a system in a package (SiP), and so forth. Although examples herein may describe computing device(s) as physical device(s), implementations are not so limited. In some examples, a computing device may include one or more of a virtual computing environment, a hypervisor, an emulation, or a virtual machine executing on one or more physical computing devices. In some examples, two or more computing devices may include a cluster, cloud, farm, or other grouping of multiple devices that coordinate operations to provide load balancing, failover support, parallel processing capabilities, shared storage resources, shared networking capabilities, or other aspects.

This specification uses the term "configured" in connection with systems and computer program components. For a system of one or more computers to be configured to perform particular operations or actions means that the system has installed on software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory storage medium for execution by, or to control the operation of, data processing apparatus. The computer storage medium can be a machine-readable storage device, a machine-readable storage substrate, a random or serial access memory device, or a combination of one or more of them. Alternatively, or in addition, the program instructions can be encoded on an artificially generated propagated signal, e.g., a machine-generated electrical, optical, or electromagnetic signal, which is generated to encode information for transmission to a suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a data communication network.

In this specification, the term "database" is used broadly to refer to any collection of data: the data does not need to be structured in any particular way, or structured at all, and it can be stored on storage devices in one or more locations. Thus, for example, the index database can include multiple collections of data, each of which may be organized and accessed differently.

Similarly, in this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Embodiments of the subject matter described in this specification can be implemented in a computing system that includes a back end component, e.g., as a data server, or that includes a middleware component, e.g., an application server, or that includes a front end component, e.g., a client computer having a graphical user interface, a web browser, or an app through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable sub combination. Moreover, although features may be described above as acting in certain combinations and even initially be claimed as such, one or more features from a claimed combination can in some cases be excised from the combination, and the claimed combination may be directed to a sub combination or variation of a sub combination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

What is claimed is:

### EMBODIMENTS

Although the present invention is defined in the claims, it should be understood that the present invention can also (alternatively) be defined in accordance with the following embodiments:
1. A method implemented by a data processing system for generating a patient specific computer-aided design (CAD) model of a mitral valve, the method comprising:
   receiving, by a data processing system, one or more digital images of a mitral valve of a patient;
   segmenting, by the data processing system, the one or more digital images to identify structures of the mitral valve;
   generating, by the data processing system, a CAD model of the mitral valve, the CAD model comprising data for modeled structures representing the identified structures of the mitral valve;
   connecting, by the data processing system in the CAD model, one or more first ones of the modeled structures to one or more second ones of the modeled structures and third ones of the modeled structures, the one or more first ones connecting to the one or more second ones and third ones at multiple locations;
   determining, by the data processing system, one or more first loading conditions for the modeled structures in the CAD model using a first hemodynamics model;
   simulating, by the data processing system, movement of the modeled structures in the CAD model based on the first loading conditions applied to the modeled structures;
   determining, by the data processing system, a specified area based on the CAD model;
   determining, by the data processing system, one or more second loading conditions using a second hemodynamics model that receives data representing the specified area as an input; and
   calibrating, by the data processing system, the CAD model by modifying a configuration of one or more first ones of the modeled structures and one or more second ones of the modeled structures in the CAD model, with the modifying based on (i) the one or more second loading conditions determined using the second hemodynamics model, and (ii) positions of the modeled structures in the CAD model based on the movement of the modeled structures in the CAD model in comparison to positions of the identified structures.
2. The method of embodiment 1, wherein segmenting the one or more digital images comprises rotating the one or more digital images to align with an annulus plane of the mitral valve.
3. The method of embodiment 1, wherein the identified structures comprise an annulus, leaflets, papillary muscles, and chordae.
4. The method of embodiment 3, wherein segmenting the one or more digital images comprises verifying a geometry of the leaflets identified in multiple digital images.
5. The method of embodiment 3, wherein the modeled structures comprise a modeled annulus, modeled leaflets, modeled papillary muscles, and modeled chordae based on the identified structures, wherein the one or more first ones comprise modeled chordae, the one or more second ones comprise the model leaflets, the one or more third ones comprise the modeled papillary muscles, and the fourth one comprises the modeled annulus.
6. The method of embodiment 5, wherein connecting the one or more first ones of the modeled structures to the one or more second ones and the one or more third ones comprises clustering the modeled chordae into circular zones on each modeled leaflet, each circular zone corresponding to a location of the multiple locations on the modeled papillary muscles.
7. The method of embodiment 5, wherein calibrating the CAD model comprises:
   determining an initial chordae length for the modeled chordae based on a thermal analysis of the CAD model in a systole condition and in a diastole condition;
   simulating movement of the modeled structures in the CAD model by iteratively determining modeled leaflet positions, orifice areas for the modeled leaflet positions, and pressures applied to the modeled leaflets, the pressures being determined using the second hemodynamics model and the orifice areas;
   determining a distance error between the simulated movement and a position of the mitral valve in the one or more digital images; and
   in response to determining that the distance error exceeds a threshold distance error, adjusting geometries of the modeled leaflets, a number of the modeled chordae, chordae insertion locations of the modeled chordae, or modeled chordae lengths to correct the distance error.
8. The method of embodiment 5, further comprising extending, by the data processing system, the modeled annulus in the CAD model to represent tissue in association with the mitral valve.
9. The method of embodiment 1, wherein the first and the second hemodynamics models comprise first and second lumped parameter hemodynamics models.
10. The method of embodiment 1, wherein generating the CAD model comprises generating modeled leaflet geometry at a diastole condition of the mitral valve, and generating modeled leaflet geometry at a systole condition of the mitral valve.
11. The method of embodiment 1, further comprising treating a patient based on performing pre-operative simulations and treatment simulations using the CAD model of the mitral valve.
12. The method of embodiment 11, wherein performing treatment simulations using the CAD model comprises placing a model of a medical device in contact with one or more of the modeled structures in the CAD model of the mitral valve.
13. The method of embodiment 12, wherein the model of the medical device comprises a model of a clip in contact with the modeled structures of the mitral valve in the CAD model.
14. The method of embodiment 1, wherein specified area comprises:
   defining two or more parallel cut planes in the CAD model;
   forming one or more connected segments;
   determining a coaptation point and a coaptation length on each of the two or more parallel cut planes based on the one or more connected segments; and
   determining the specified area based on the coaptation length and a distance between two parallel cut planes of the two or more parallel cut planes.
15. The method of embodiment 1, wherein the first and the second hemodynamics models comprise parameters determined by minimizing a difference between outputs of the first and the second hemodynamics models and corresponding measurements from the patient and literature data.
16. The method of embodiment 1, further comprising generating clinical metrics for use in assessment of mitral valve regurgitation in pre-operative and post-operative states of treated patients, wherein the clinical metrics include left atrial pressure, transmitral pressure gradient, and mitral valve regurgitant volume.
17. A computer system for generating a patient specific computer-aided design (CAD) model of a mitral valve, the computer system comprising:
   one or more processors;
   and a memory storing instructions that when executed by the at least one processor cause the at least one processor to perform operations comprising:
      receiving one or more digital images of a mitral valve of a patient;
      segmenting the one or more digital images to identify structures of the mitral valve;
      generating a CAD model of the mitral valve, the CAD model comprising data for modeled structures representing the identified structures of the mitral valve;
      connecting, in the CAD model, one or more first ones of the modeled structures to one or more second ones of the modeled structures and third ones of the modeled structures, the one or more first ones connecting to the one or more second ones and third ones at multiple locations;
      determining one or more first loading conditions for the modeled structures in the CAD model using a first hemodynamics model;
      simulating movement of the modeled structures in the CAD model based on the first loading conditions applied to the modeled structures;
      determining a specified area based on the CAD model;
      determining one or more second loading conditions using a second hemodynamics model that receives data representing the specified area as an input; and
      calibrating the CAD model by modifying a configuration of one or more first ones of the modeled structures and one or more second ones of the modeled structures in the CAD model, with the modifying based on (i) the one or more second loading conditions determined using the second hemodynamics model, and (ii) positions of the modeled structures in the CAD model based on the movement of the modeled structures in the CAD model in comparison to positions of the identified structures.
18. The computer system of embodiment 17, wherein calibrating the CAD model comprises:
   determining an initial chordae length for the modeled chordae based on a thermal analysis of the CAD model in a systole condition and in a diastole condition;
   simulating movement of the modeled structures in the CAD model by iteratively determining modeled leaflet positions, orifice areas for the modeled leaflet positions, and pressures applied to the modeled leaflets, the pressures being determined using the second hemodynamics model and the orifice areas;
   determining a distance error between the simulated movement and a position of the mitral valve in the one or more digital images; and
   in response to determining that the distance error exceeds a threshold distance error, adjusting geometries of the modeled leaflets, a number of the modeled chordae, chordae attachment locations of the modeled chordae, or modeled chordae lengths to correct the distance error.
19. One or more non-transitory, computer readable storage media storing instructions for generating a patient specific computer-aided design (CAD) model of a mitral valve, the instructions when executed by at least one processor cause the at least one processor to perform operations comprising:
   receiving one or more digital images of a mitral valve of a patient;
   segmenting the one or more digital images to identify structures of the mitral valve;
   generating a CAD model of the mitral valve, the CAD model comprising data for modeled structures representing the identified structures of the mitral valve;
   connecting, in the CAD model, one or more first ones of the modeled structures to one or more second ones of the modeled structures and third ones of the modeled structures, the one or more first ones connecting to the one or more second ones and third ones at multiple locations;
   determining one or more first loading conditions for the modeled structures in the CAD model using a first hemodynamics model;
   simulating movement of the modeled structures in the CAD model based on the first loading conditions applied to the modeled structures;
   determining a specified area based on the CAD model;
   determining one or more second loading conditions using a second hemodynamics model that receives data representing the specified area as an input; and
   calibrating the CAD model by modifying a configuration of one or more first ones of the modeled structures and one or more second ones of the modeled structures in the CAD model, with the modifying based on (i) the one or more second loading conditions determined using the second hemodynamics model, and (ii) positions of the modeled structures in the CAD model based on the movement of the modeled structures in the CAD model in comparison to positions of the identified structures.
20. The one or more non-transitory, computer readable storage media of embodiment 17, wherein calibrating the CAD model comprises:
   determining an initial chordae length for the modeled chordae based on a thermal analysis of the CAD model in a systole condition and in a diastole condition;
   simulating movement of the modeled structures in the CAD model by iteratively determining modeled leaflet positions, orifice areas for the modeled leaflet positions, and pressures applied to the modeled leaflets, the pressures being determined using the second hemodynamics model and the orifice areas;
   determining a distance error between the simulated movement and a position of the mitral valve in the one or more digital images; and
   in response to determining that the distance error exceeds a threshold distance error, adjusting geometries of the modeled leaflets, a number of the modeled chordae, chordae attachment locations of the modeled chordae, or modeled chordae lengths to correct the distance error.

## Claims

1. A method implemented by a data processing system for generating a patient specific computer-aided design (CAD) model of a mitral valve, the method comprising:
receiving, by a data processing system, one or more digital images of a mitral valve of a patient;
segmenting, by the data processing system, the one or more digital images to identify structures of the mitral valve;
generating, by the data processing system, a CAD model of the mitral valve, the CAD model comprising data for modeled structures representing the identified structures of the mitral valve;
connecting, by the data processing system in the CAD model, one or more first ones of the modeled structures to one or more second ones of the modeled structures and third ones of the modeled structures, the one or more first ones connecting to the one or more second ones and third ones at multiple locations;
determining, by the data processing system, one or more first loading conditions for the modeled structures in the CAD model using a first hemodynamics model;
simulating, by the data processing system, movement of the modeled structures in the CAD model based on the first loading conditions applied to the modeled structures;
determining, by the data processing system, a specified area based on the CAD model;
determining, by the data processing system, one or more second loading conditions using a second hemodynamics model that receives data representing the specified area as an input; and
calibrating, by the data processing system, the CAD model by modifying a configuration of one or more first ones of the modeled structures and one or more second ones of the modeled structures in the CAD model, with the modifying based on (i) the one or more second loading conditions determined using the second hemodynamics model, and (ii) positions of the modeled structures in the CAD model based on the movement of the modeled structures in the CAD model in comparison to positions of the identified structures.

2. The method of claim 1, wherein segmenting the one or more digital images comprises rotating the one or more digital images to align with an annulus plane of the mitral valve.

3. The method of any preceding claim, wherein the identified structures comprise an annulus, leaflets, papillary muscles, and chordae.

4. The method of claim 3, wherein segmenting the one or more digital images comprises verifying a geometry of the leaflets identified in multiple digital images, and/or wherein the modeled structures comprise a modeled annulus, modeled leaflets, modeled papillary muscles, and modeled chordae based on the identified structures, wherein the one or more first ones comprise modeled chordae, the one or more second ones comprise the model leaflets, the one or more third ones comprise the modeled papillary muscles, and the fourth one comprises the modeled annulus.

5. The method of claim 4, wherein connecting the one or more first ones of the modeled structures to the one or more second ones and the one or more third ones comprises clustering the modeled chordae into circular zones on each modeled leaflet, each circular zone corresponding to a location of the multiple locations on the modeled papillary muscles.

6. The method of claim 4, wherein calibrating the CAD model comprises:
determining an initial chordae length for the modeled chordae based on a thermal analysis of the CAD model in a systole condition and in a diastole condition;
simulating movement of the modeled structures in the CAD model by iteratively determining modeled leaflet positions, orifice areas for the modeled leaflet positions, and pressures applied to the modeled leaflets, the pressures being determined using the second hemodynamics model and the orifice areas;
determining a distance error between the simulated movement and a position of the mitral valve in the one or more digital images; and
in response to determining that the distance error exceeds a threshold distance error, adjusting geometries of the modeled leaflets, a number of the modeled chordae, chordae insertion or attachment locations of the modeled chordae, or modeled chordae lengths to correct the distance error.

7. The method of claim 4, further comprising extending, by the data processing system, the modeled annulus in the CAD model to represent tissue in association with the mitral valve.

8. The method of claim 1, wherein the first and the second hemodynamics models comprise first and second lumped parameter hemodynamics models.

9. The method of claim 1, wherein generating the CAD model comprises generating modeled leaflet geometry at a diastole condition of the mitral valve, and generating modeled leaflet geometry at a systole condition of the mitral valve.

10. The method of claim 1, further comprising treating a patient based on performing pre-operative simulations and treatment simulations using the CAD model of the mitral valve, optionally wherein performing treatment simulations using the CAD model comprises placing a model of a medical device in contact with one or more of the modeled structures in the CAD model of the mitral valve, and optionally wherein the model of the medical device comprises a model of a clip in contact with the modeled structures of the mitral valve in the CAD model.

11. The method of claim 1, wherein specified area comprises:
defining two or more parallel cut planes in the CAD model;
forming one or more connected segments;
determining a coaptation point and a coaptation length on each of the two or more parallel cut planes based on the one or more connected segments; and
determining the specified area based on the coaptation length and a distance between two parallel cut planes of the two or more parallel cut planes.

12. The method of claim 1, wherein the first and the second hemodynamics models comprise parameters determined by minimizing a difference between outputs of the first and the second hemodynamics models and corresponding measurements from the patient and literature data.

13. The method of claim 1, further comprising generating clinical metrics for use in assessment of mitral valve regurgitation in pre-operative and post-operative states of treated patients, wherein the clinical metrics include left atrial pressure, transmitral pressure gradient, and mitral valve regurgitant volume.

14. A computer system for generating a patient specific computer-aided design (CAD) model of a mitral valve, the computer system comprising:
one or more processors;
and a memory storing instructions that when executed by the at least one processor cause the at least one processor to perform operations comprising the method of any one of claims 1 to 13.

15. One or more non-transitory, computer readable storage media storing instructions for generating a patient specific computer-aided design (CAD) model of a mitral valve, the instructions when executed by at least one processor cause the at least one processor to perform operations comprising the method of any one of claims 1 to 13.
